# EUROPEAN PATENT APPLICATION

(11) **EP 3 034 600 A1**
(43) Date of publication of application: **22.06.2016**
(21) Application number: 14198283.5
(22) Date of filing: 16.12.2014
(51) Int. Cl.: C12M 1/00, C12N 1/04, C12N 1/14, A01N 63/04

(54) **Method for prolonging the viability of fungal spores in liquid formulations**

(71) Applicant: Bayer CropScience Biologics GmbH, 23999 Malchow/Poel (DE); Bayer CropScience AG, 40789 Monheim am Rhein (DE)
(72) Inventor: The designation of the inventor has not yet been filed
(74) Representative: BIP Patents

(57) **Abstract**

The present invention relates to a method for prolonging spore viability of fungal spores present in a liquid formulation, comprising (a) packaging said formulation in a suitable container; (b) reducing oxygen exposure of the spores in said formulation as compared to oxygen exposure of said spores when said formulation is in contact with air; and (c) closing or sealing said container. The invention further relates to a closed container comprising a liquid formulation within said container, said formulation comprising fungal spores, wherein the spores in said formulation have reduced oxygen exposure as compared to oxygen exposure of said spores when said formulation is in contact with air.

## Description

In agriculture, plant protection products are commonly used in order to combat plant diseases, pests or weed plants or to strengthen the physiological functions of plants. Usually, these products are marketed in sealed packages. Known packages include those which comprise a container of the product, such as for example a bottle, a tube or the like, which is closed within an external protective enclosure such as for example a sachet, a case or the like, which can be removed at the time of use.

Biological control agents (BCA) usually comprise a fungal or bacterial microorganism and can be used e. g. as biological fungicide, pesticide or herbicide or for promoting plant health. Spore-based fungal products are applied to the soil, plant surfaces or seeds. The spores germinate, grow and protect a treated area.

During the last years the demand for biological control agents as an alternative to synthetic chemical plant protection agents has increased. Such biological control agents are usually available as solid formulation, e. g. as wettable powder (WP) or water dispersible granules (WG). Only few liquid formulations are available due to their restrictions in storage stability. It is therefore desirable to have a liquid formulation at hand which does not have the mentioned disadvantages and which has an increased shelf life both at ambient temperature and at high or low temperatures.

Accordingly, it is one object of the present invention to provide means to increase the storage stability of liquid formulations comprising fungal-based biological control agents. A further object of the invention is to increase viability of the spores comprised in such formulation.

Accordingly, the present invention relates to a method for prolonging viability of fungal spores present in a liquid formulation, comprising (a) packaging said formulation in a suitable container; (b) reducing oxygen exposure of the spores in said formulation as compared to oxygen exposure of said spores when said formulation is in contact with air; and (c) closing or sealing said container.

In general, fungal spores can be prepared through liquid or solid fermentation as known in the art or referred to elsewhere in this application. Subsequently, the spores are separated from the culture substrate. In order to conserve the spores, they are dried via e. g. freeze-drying, vacuum drying or spray drying after separation and before formulation. Where necessary, after the drying, the spores are combined through blending, spraying, mixing, and/or extruding with one or more inert solid carriers that can include, but are not limited to, clay, bran, lactose, cellulose, vermiculite, or sawdust. The liquid formulation is then packaged in accordance with the method of the present invention.

Prolonging spore viability in connection with the present invention refers to prolonging the time span a spore can remain viable and is still able to germinate after a certain storage time and preferably also following application of the product, e.g. spray application or soil drench, of the formulated spores.. This prolonged viability is in comparison with that of spores which are exposed to higher contents of oxygen in the atmosphere being in contact with the formulation, preferably in comparison with spore viability of spores exposed to air having an oxygen content of about 21%. As shown in the examples, a higher reduction of oxygen compared to a lower reduction of oxygen still has the desired effect, even if a lower reduction also already results in prolonged spore viability.
The time span by that spore viability is prolonged according to the present invention varies depending on the temperature as also shown in the appended examples. Furthermore, storage time of the formulation plays a role for viability. For example, in the present invention at least 10% more spores (value based on the initial spore viability measured directly before storage of the formulation) remain viable after storage of 8 weeks at 30°C when permeation of oxygen through the packaging material is reduced (here by using an oxygen-impermeable packaging material) as compared to lower reduction of oxygen contained in the atmosphere surrounding the formulation (by using an oxygen-permeable packaging material where oxygen can enter but is consumed by the formulation at a faster rate than re-entry thus resulting in a lower oxygen content as compared to the atmospheric oxygen content).

The method of the present invention results in a prolonged shelf life/storage stability of a liquid formulation comprising fungal spores. Shelf life may be defined differently. For the purpose of the present invention only, shelf life is defined as the time a formulation may be stored so that still 20% of the spores contained therein remain viable. After this time, shelf life has expired. Looking at example 2c, shelf life of the formulation not flushed with nitrogen has expired after storage for 6 months at 30°C whereas the formulation flushed with nitrogen still shows 71% viability.

In one embodiment, said spore viability at a certain point in time is prolonged by at least 10%, as compared to that of a formulation where the surrounding oxygen content is not reduced. For example, spore viability is prolonged by at least 10% after storage at 20°C for 6 months. In other words, at least 10% more spores remain viable after storage at 20°C for 6 months. In another embodiment, said spore viability at a certain point in time is prolonged by at least 20%, as compared to that of a formulation where the surrounding oxygen content is less reduced. For example, spore viability is prolonged by at least 20% after storage at 25°C for 6 months. In another embodiment, said spore viability at a certain point in time is prolonged by at least 20%, as compared to that of a formulation where the surrounding oxygen content is less reduced. For example, spore viability is prolonged by at least 30%, preferably at least 40%, more preferably at least 50% after storage at 30°C for 6 months. It is to be understood that in some embodiments, said spore viability is a combination of more than one or even all of the embodiments described above. Spore viability is also dependent on the fungal species used and therefore may vary accordingly.

It has been observed that the advantageous prolongation in spore viability and product stability after storage is particularly apparent when measured after storage at elevated temperatures, defined herein as temperatures greater than 25 °C. The method according to the invention to prolong spore viability and product stability after storage at ambient as well as elevated temperatures is particularly advantageous for application of liquid formulations comprising fungal-based BCAs in warmer climatic zones with less need for refrigeration during transport and storage as refrigeration is currently not common practice for crop protection products and equally would be a significant hurdle or even prohibitive due to the costs involved.

Spore viability is measured by removing a representative sample of a liquid formulation comprising suspended spores after a storage period and determining the ratio of still viable spores to not viable spores on a growth medium suitable for the particular organism. The ratio of still germinating spores and the total determined spores, expressed as percentage is the expression of the viability of the formulated fungus.

Fungal spores include sexually (e. g. oospores, zygospores or ascospores) and asexually (e. g. conidia and chlamydospores, but also uredospores, teleutospores and ustospores) formed spores.

A liquid formulation in connection with the present invention includes fungal spores in suspension. In order to prevent sedimentation and to ensure equal distribution of the spores within the liquid, the formulation may comprise additional formulants such as thickeners, as will be described in more detail further below.

A suitable container may be any container which is sealable or which at least can be closed, whereby sealable containers are preferred. Many kinds of material allow for the production of containers suitable in the present invention, e. g. glass, metal (such as aluminum and tin plate), plastic (such as thermoplasts like HDPE (high-density polyethylene), PA (polyamide), EVOH (ethylene-vinylalcolhol copolymer), PET (polyethylene terephthalate), PP (polypropylene), biopolymers, composite materials (such as cellulose materials like paper, cardboard, corrugated paper lined with barrier materials), barrier materials such as Coex (HDPE/PA, HDPE/EVOH) for hollow containers, and multilayer films and foil laminates (such as aluminum lined PE, PET, PA, EVOH, LDPE (low density polyethylene), PVC (polyvinyl chloride), EVA (ethylene-vinyl acetate) and OPP (oriented PP).

The container used in the present invention may be in various forms (such as bottle, bag (stand-up pouch, horizontal tubular bag), can and pot) and have various shapes with sealable and non-sealable closures. Closures can be of various kinds, such as breathable, preferably non-breathable and more preferably sealed. In some cases it may be preferable that the closure is hermetically sealed.

Breathable closings range from single layer polymers to multi-layered composites of papers, films, foils and coatings and include foams (such as PE) and membranes.

Non-breathable closings include induction seal liners. Induction sealing, otherwise known as cap sealing, is a non-contact heating process that accomplishes the hermetic sealing of a container with a closure that includes a heat-sealable foil laminate. The typical induction inner seal begins as a multi-laminate liner inside a closure. It consists of a layer of pulp board, a layer of wax, aluminum foil and a layer of polymer that is compatible with the bottle material and capable of heat sealing to the lip of the container. This sealing process takes place after the container has been filled and capped.

Reducing oxygen exposure refers to reducing the oxygen content of the atmosphere surrounding the liquid formulation. Such reduction may be effected by removing oxygen from the surrounding atmosphere in contact with the formulation. The oxygen content of the surrounding atmosphere is thereby reduced compared to the atmospheric oxygen content of ca. 21 %, preferably by at least 30%, preferably at least 50%, more preferably at least 70%, 80%, 90% or even at least 95%. It is most preferred that the surrounding atmosphere in contact with the liquid formulation - if any - is essentially free of oxygen. In this regard, essentially free of oxygen relates to oxygen contents of less than 5%, preferably 2% or less, even more preferably 1% or less, such as 0.5% or even 0.2% 0.1% or 0%.

As shown in the examples, reducing oxygen exposure of a liquid formulation could markedly reduce the loss of spore viability during storage which results in an increased storage stability and a prolonged shelf life of the respective formulation.

In a preferred embodiment, the container is with reduced or without head space.

Head space is the space within a sealed container which is not filled with product, in particular liquid formulation. A reduced head space refers to a volume of head space which is less than 30% of the volume of the liquid formulation, preferably less than 20%, more preferably less than 10% and even more preferably less than 5%.

In some cases, it may be possible to produce containers comprising the formulation which are essentially without head space, meaning a head space of less than 5%, preferably less than 2%, more preferably less than 1% of the volume of the liquid formulation comprised in a container.

A small or no head space is preferred in case re-suspension by shaking the liquid formulation is not necessary. On the other hand, a certain head space is needed for those cases where the fungal spores may sediment during storage and need to be re-suspended prior to use. In such cases, at least 15% head space (of the total volume of the container) is generally needed for enabling for re-suspension of the spores. In general, a suitable range of minimal head space lies between 15 and 18%, but may vary depending on the formulation.

In another preferred embodiment, the container is an airtight system, hermetically sealed with a container material impermeable to air. This can be combined with a reduced head space or no head space of the container.

In one preferred embodiment, said container reduces or prevents diffusion of oxygen. This feature, in this embodiment, is equivalent to step (b) of reducing oxygen exposure because already reducing diffusion of oxygen result in less oxygen exposure as compared to an open vessel where oxygen is freely diffusing and thus the initial oxygen content is retained. This is due to the fact that the formulation comprising living material consumes oxygen and therefore, in a system with reduced or no diffusion, the oxygen content will inevitably decrease.

In another preferred embodiment, reducing oxygen exposure in step (b) is effected by reducing oxygen in the head space of said container.

Reducing oxygen in the head space of a container may be effected in several ways.

In one embodiment, reducing oxygen exposure in step (b) is effected by evacuating said container.

Evacuating may take place before or after filling said container with the liquid formulation. Applicability of this technique depends on the shape and material of the container.

Vacuum packaging involves packaging the product in containers with low or no oxygen permeability and sealing it after evacuating the air. Using this technique oxygen levels may be reduced to less than 1 %. The barrier properties of the container material restrict entry of oxygen from outside.

In another embodiment, reducing oxygen exposure in step (b) is effected by evacuating said container and subsequently refilling with an (inert) gas or mixtures of (inert) gases.
This technology is also referred to as compensated vacuum. The compensated vacuum technique removes the air inside by pulling a vacuum on the atmosphere inside the package and then breaking the vacuum with the desired gas mixture. Since the replacement of the air is accomplished in a two-step process, the speed of operation is slower than the gas flush technique. However, since the air is removed by vacuum and not simply diluted, the efficiency of this process with respect to residual oxygen levels is better.

The refilling is preferably done immediately after evacuating in order to avoid penetration of air into the evacuated container.

Gases suitable for use in the present invention are those which do not adversely influence spore viability. Preferably, they are inert.
Suitable gases include but are not limited to hydrogen, nitrogen, helium, neon, argon, krypton, xenon, radon, carbon dioxide, nitrous oxide, hydrogen sulfide, lower alkane or halo alkane or mixtures thereof, preferably nitrogen, argon, carbon dioxide or mixtures thereof.

Two techniques which may be used in the present invention are gas-flushing and compensated vacuum. In gas-flushing the desired gas mixture is instilled in quantity into the packaging, pushing out the air, whereas in compensated vacuum the air is removed and the desired gas mixture then instilled.

In a more preferred embodiment, evacuating said container and subsequent refilling with an (inert) gas or mixtures of (inert) gases is repeated at least twice. Depending on the success, i.e. the percentage of oxygen remaining in the container, this step may be repeated until the desired oxygen content is achieved.

In another preferred embodiment, oxygen exposure in step (b) is reduced by flushing said head space with a suitable, preferably an inert gas or a mixture of such gases comprising less oxygen compared to air or no oxygen. In this way, a modified atmosphere is created. Generally, said flushing may be effected prior to or after filling the container with the liquid formulation. However, it is preferred that the flushing step is done after filling of the container with the liquid formulation.

In a more preferred embodiment, said gas is nitrogen. Nitrogen flushing is a preservation method already used to protect packaged foods and chemical plant protection agents. Nitrogen flushing and sealing machines are used to force the regular air out of the packaging and introduce nitrogen gas into the packaging.

In a preferred embodiment said container reduces or prevents diffusion of oxygen.

Reducing or preventing diffusion of oxygen can be influenced by one or more materials used in producing the container. Either the container consists of a material which prevents or reduces diffusion. Alternatively, the container may be covered, on the inside or the outside, with a film reducing or preventing diffusion of oxygen.

In another preferred embodiment, said formulation is essentially free of water. Such formulation is e. g. described in European patent application EP14187473.

In brief, a preferred formulation essentially free of water is a (liquid) composition comprising at least one biological control agent, wherein said biological control agent is spores of a spore forming fungus, a polyether-modified trisiloxane and fumed silica or precipitated silica.
If water is present, such water mainly comes from water in the dried spore powder or traces of water in the other components of the formulation. Accordingly, the water concentration highly depends on the amount of spore powder mixed into the liquid formulation. The higher the amount of spore powder the higher the water content may be. Water concentrations of between 0.3 and 5 % are possible due to these facts, which range would then fall within the definition of "essentially free of water". The amount of spore powder in the liquid formulation also depends on the application so that a composition for use in nematode control may need a higher spore concentration than one for use for increasing plant health in general. Accordingly, exemplary water concentrations include 1 %, 2%, 3%, 4% and 5%.

The invention also relates to a closed container comprising a liquid formulation within said container, said formulation comprising fungal spores, wherein the spores in said formulation have reduced oxygen exposure as compared to oxygen exposure of said spores when said formulation is in contact with air.

A wide range of fungal organisms can produce spores for use in the method of the present invention.

If the fungal strain producing spores has a fungicidal effect, it may be selected from
B2.1 *Coniothyrium minitans,* in particular strain CON/M/91-8 (Accession No. DSM-9660; e.g. Contans ® from Prophyta); B2.3 *Microsphaeropsis ochracea*, *in particular* strain P130A (ATCC deposit 74412); B2.5 *Trichoderma spp.,* including *Trichoderma atroviride*, strain SC1 described in International Application No. PCT/IT2008/000196); B2.6 *Trichoderma harvianum rifai* strain KRL-AG2 (also known as strain T-22, /ATCC 208479, e.g. PLANTSHIELD T-22G, Rootshield®, and TurfShield from BioWorks, US); B2.7 *Arthrobotrys dactyloides;* B2.8 *Arthrobotrys oligospora*; B2.9 *Arthrobotrys superba;* B2.10 *Aspergillus flavus,* e. g. strain NRRL 21882 (e.g. Afla-Guard® from Syngenta); B2.11 *Aspergillus flavus,* e. g. strain AF36 (e.g. AF36 from Arizona Cotton Research and Protection Council, US); B2.14 *Gliocladium roseum*, e. g. strain 321U from Adjuvants Plus; B2.15 *Phlebiopsis* (or *Phlebia* or *Peniophora*) *gigantea,* in particular strain VRA 1835 (ATCC 90304); B2.16 *Phlebiopsis* (or *Phlebia* or *Peniophora*) *gigantea,* in particular strain VRA 1984 (DSM16201); B2.17 *Phlebiopsis* (or *Phlebia* or *Peniophora*) *gigantea,* in particular strain VRA 1985 (DSM16202); B2.18 *Phlebiopsis* (or *Phlebia* or *Peniophora*) *gigantea,* in particular strain VRA 1986 (DSM16203); B2.19 *Phlebiopsis* (or *Phlebia* or *Peniophora*) *gigantea,* in particular strain FOC PG B20/5 (IMI390096); B2.20 *Phlebiopsis* (or *Phlebia* or *Peniophora*) *gigantea,* in particular strain FOC PG SP log6 (IMI390097); B2.21 *Phlebiopsis* (or *Phlebia* or *Peniophora*) *gigantea,* in particular strain FOC PG SP log5 (IMI390098); B2.22 *Phlebiopsis* (or *Phlebia* or *Peniophora*) *gigantea,* in particular strain FOC PG BU3 (IMI390099); B2.23 *Phlebiopsis* (or *Phlebia* or *Peniophora*) *gigantea,* in particular strain FOC PG BU4 (IMI390100); B2.24 *Phlebiopsis* (or *Phlebia* or *Peniophora*) *gigantea,* in particular strain FOC PG 410.3 (IMI390101); B2.25 *Phlebiopsis* (or *Phlebia* or *Peniophora*) *gigantea,* in particular strain FOC PG 97/1062/116/1.1 (IMI390102); B2.26 *Phlebiopsis* (or *Phlebia* or *Peniophora*) *gigantea,* in particular strain FOC PG B22/SP1287/3.1 (IMI390103); B2.27 *Phlebiopsis* (or *Phlebia* or *Peniophora*) *gigantea,* in particular strain FOC PG SH1 (IMI390104); B2.28 *Phlebiopsis* (or *Phlebia* or *Peniophora*) *gigantea,* in particular strain FOC PG B22/SP1190/3.2 (IMI390105) (B2.15 to B2.28: e.g. Rotstop® from Verdera and FIN, PG-Agromaster®, PG-Fungler®, PG-IBL®, PG-Poszwald®, and Rotex® from e-nema, DE); B2.29 *Pythium oligandrum*, strain DV74 or M1 (ATCC 38472; e.g. Polyversum from Bioprepraty, CZ); B2.35 *Talaromyces flavus,* e. g. strain VII7b; B2.36 *Trichoderma asperellum*, e. g. strain ICC 012 from Isagro; B2.37 *Trichoderma asperellum*, strain SKT-1 (e.g. ECO-HOPE® from Kumiai Chemical Industry); B2.38 *Trichoderma atroviride*, e. g. strain CNCM I-1237 (e.g. Esquive® WP from Agrauxine, FR); B2.39 *Trichoderma atroviride*, strain no. V08/002387; B2.40 *Trichoderma atroviride*, strain NMI no. V08/002388; B2.41 *Trichoderma atroviride*, strain NMI no. V08/002389; B2.42 *Trichoderma atroviride*, strain NMI no. V08/002390; B2.43 *Trichoderma atroviride,* strain LC52 (e.g. Tenet by Agrimm Technologies Limited); B2.44 *Trichoderma atroviride,* strain ATCC 20476 (IMI 206040); B2.45 *Trichoderma atroviride,* strain T11 (IMI352941/ CECT20498); B2.46 *Trichoderma harmatum;* B2.47 *Trichoderma harzianum*; B2.48 *Trichoderma harzianum rifai T39* (e.g. Trichodex® from Makhteshim, US); B2.49 *Trichoderma harzianum*, in particular, strain KD (e.g. Trichoplus from Biological Control Products, SA (acquired by Becker Underwood)); B2.50 *Trichoderma harzianum*, strain ITEM 908 (e.g. Trianum-P from Koppert); B2.51 *Trichoderma harzianum*, strain TH35 (e.g. Root-Pro by Mycontrol); B2.52 *Trichoderma virens* (also known as *Gliocladium virens*), in particular strain GL-21 (e.g. SoilGard 12G by Certis, US); B2.53 *Trichoderma viride,* e. g. strain TV1(e.g. Trianum-P by Koppert); B2.54 *Ampelomyces quisqualis,* in particular strain AQ 10 (e.g. AQ 10® by IntrachemBio Italia); B2.62 *Chaetomium cupreum* (e.g. BIOKUPRUM TM by AgriLife); B2.63 *Chaetomium globosum* (e.g. Rivadiom by Rivale); B2.64 *Cladosporium cladosporioides,* e. g. strain H39 (by Stichting Dienst Landbouwkundig Onderzoek); B2.66 *Dactylaria candida;* B2.67 *Dilophosphora alopecuri* (e.g. Twist Fungus); B2.68 *Fusarium oxysporum,* strain Fo47 (e.g. Fusaclean by Natural Plant Protection); B2.69 *Gliocladium catenulatum* (Synonym: *Clonostachys rosea f. catenulate*), e. g. strain J1446 (e.g. Prestop ® by AgBio Inc. and also e.g. Primastop®
by Verdera Oy); B2.70 *Lecanicillium lecanii* (formerly known as *Verticillium lecanii*) *conidia, e. g.* of strain KV01 (e.g. Vertalec® by Koppert/Arysta); B2.71 *Penicillium vermiculatum;* B2.75 *Trichoderma atroviride,* strain SKT-1 (FERM P-16510); B2.76 *Trichoderma atroviride,* strain SKT-2 (FERM P-16511); B2.77 *Trichoderma atroviride,* strain SKT-3 (FERM P-17021); B2.78 *Trichoderma gamsii* (formerly *T. viride*), strain ICC080 (IMI CC 392151 CABI, e.g. BioDerma by AGROBIOSOL DE MEXICO, S.A. DE C.V.); B2.79*Trichoderma harzianum*, strain DB 103 (e.g. T-Gro 7456 by Dagutat Biolab); B2.80 *Trichoderma polysporum,* strain IMI 206039 (e.g. Binab TF WP by BINAB Bio-Innovation AB, Sweden); B2.81 *Trichoderma stromaticum* (e.g. Tricovab by Ceplac, Brazil); B2.83 *Ulocladium oudemansii,* in particular strain HRU3 (e.g. Botry-Zen® by Botry-Zen Ltd, NZ); B2.84 *Verticillium albo-atrum* (formerly *V. dahliae),* strain WCS850 (CBS 276.92; e.g. Dutch Trig by Tree Care Innovations); B2.86 *Verticillium chlamydosporium*; B2.87 mixtures of *Trichoderma asperellum* strain ICC 012 and *Trichoderma gamsii* strain ICC 080 (product known as e.g. BIO-TAM™ from Bayer CropScience LP, US); and 2.88 Simplicillium lanosoniveum.

In a preferred embodiment, the fungal strain having fungicidal activity is selected from

*Coniothyrium minitans,* in particular strain CON/M/91-8 (Accession No. DSM-9660) (available as Contans ® from Prophyta, DE); *Microsphaeropsis ochracea* strain P130A (ATCC 74412); *Aspergillus flavus,* strain NRRL 21882 (available as Afla-Guard® from Syngenta) and strain AF36 (available as AF36 from Arizona Cotton Research and Protection Council, US); *Gliocladium roseum*, strain 321U from Adjuvants Plus; *Phlebiopsis* (or *Phlebia* or *Peniophora*) *gigantea,* in particular the strains VRA 1835 (ATCC 90304), VRA 1984 (DSM16201), VRA 1985 (DSM16202), VRA 1986 (DSM16203), FOC PG B20/5 (IMI390096), FOC PG SP log6 (IMI390097), FOC PG SP log5 (IMI390098), FOC PG BU3 (IMI390099), FOC PG BU4 (IMI390100), FOC PG 410.3 (IMI390101), FOC PG 97/1062/116/1.1 (IMI390102), FOC PG B22/SP1287/3.1 (IMI390103), FOC PG SH1 (IMI390104), FOC PG B22/SP1190/3.2 (IMI390105) (available as Rotstop® from Verdera and FIN, PG-Agromaster®, PG-Fungler®, PG-IBL®, PG-Poszwald®, and Rotex® from e-nema, DE); *Pythium oligandrum*, strain DV74 or M1 (ATCC 38472) (available as Polyversum from Bioprepraty, CZ); *Scleroderma citrinum*; *Talaromyces flavus,* strain VII7b; *Ampelomyces quisqualis,* in particular strain AQ 10 (available as AQ 10® by IntrachemBio Italia); *Gliocladium catenulatum* (Synonym: Clonostachys rosea f. catenulate) strain J1446 (available as Prestop® by AgBio Inc. and also available as Primastop® by Verdera Oy) and *Cladosporium cladosporioides*, e. g. strain H39 (by Stichting Dienst Landbouwkundig Onderzoek).

In an even more preferred embodiment, the fungal strain having fungicidal activity is selected from *Coniothyrium minitans,* in particular strain CON/M/91-8 (Accession No. DSM-9660) (available as Contans ® from Prophyta, DE);, *Talaromyces flavus,* strain VII7b; *Cladosporium cladosporioides*, e. g. strain H39 (by Stichting Dienst Landbouwkundig Onderzoek); and Simplicillium lanosoniveum.

If the fungal strain producing spores has an insecticidal effect, it may be selected from
C2.3 *Beauveria bassiana, e. g.* strain ATCC 74040 (e.g. Naturalis® from Intrachem Bio Italia); C2.4 *Beauveria bassiana* strain GHA (Accession No. ATCC74250; e.g. BotaniGuard Es and Mycontrol-O from Laverlam International Corporation); C2.5 *Beauveria bassiana* strain ATP02 (Accession No. DSM 24665); C2.6 *Beauveria bassiana* strain CG 716 (e.g. BoveMax® from Novozymes); C2.7 *Hirsutella citriformis*; C2.8 *Hirsutella thompsonii* (with some strains e.g. Mycohit and ABTEC from Agro Bio-tech Research Centre, IN); C2.9 *Lecanicillium lecanii* (formerly known as *Verticillium lecanii),* in particular *conidia* of strain KV01 (e.g. Mycotal® and Vertalec® from Koppert/Arysta); C2.10 *Lecanicillium lecanii* (formerly known as *Verticillium lecanii*) *conidia* of strain strain DAOM198499; C2.11 *Lecanicillium lecanii* (formerly known as *Verticillium lecanii*) *conidia* of strain strain DAOM216596; C2.12 *Lecanicillium muscarium* (formerly *Verticillium lecanii),* in particular strain VE 6 / CABI(=IMI) 268317/ CBS102071/ ARSEF5128; C2.13 *Metarhizium anisopliae,* in particular strain F52 (DSM3884/ ATCC 90448; e.g. BIO 1020 by Bayer CropScience and also e.g. Met52 by Novozymes); C2.14 *M. anisopliae var acridum* (e.g. GreenGuard by Becker Underwood, US); C2.15 *M. anisopliae var acridum* isolate IMI 330189 (ARSEF7486; e.g. Green Muscle by Biological Control Products); C2.16 *Nomuraea rileyi*; C2.17 *Paecilomyces fumosoroseus* (new: *Isaria fumosorosea*), in particular strain apopka 97 (e.g. PreFeRal® WG from Biobest); C2.18 *Paecilomyces fumosoroseus* (new: *Isaria fumosorosea*) strain FE 9901 (e.g. NoFly® from Natural Industries Inc., a Novozymes company); C2.19 *Aschersonia aleyrodis;* C2.20 *Beauveria brongniartii* (e.g. Beaupro from Andermatt Biocontrol AG); *C2.24 Metarhizium flavoviride*; and C2.25 *Mucor haemelis* (e.g. BioAvard from Indore Biotech Inputs & Research).

In a more preferred embodiment, fungal strains having an insecticidal effect may be selected from Beauveria bassiana, strain ATCC 74040 (available as Naturalis® from Intrachem Bio Italia), strain GHA (Accession No. ATCC74250) (available as BotaniGuard Es and Mycontrol-O from Laverlam International Corporation), strain ATP02 (Accession No. DSM 24665), strain CG 716 (available as BoveMax® from Novozymes); Hirsutella citriformis; Hirsutella thompsonii (with some strains available as Mycohit and ABTEC from Agro Bio-tech Research Centre, IN); Lecanicillium lecanii (formerly known as Verticillium lecanii) conidia of strain KV01 (available as Mycotal® and Vertalec® from Koppert/Arysta); Lecanicillium lecanii (formerly known as Verticillium lecanii) conidia of strain strain DAOM198499; Lecanicillium lecanii (formerly known as Verticillium lecanii) conidia of strain DAOM216596; Lecanicillium muscarium (formerly Verticillium lecanii), strain VE 6 / CABI(=IMI) 268317/ CBS102071/ ARSEF5128; Metarhizium anisopliae, strain F52 (DSM3884/ ATCC 90448) (available as BIO 1020 by Bayer CropScience and also available as Met52 by Novozymes); M. anisopliae var acridum (available as GreenGuard by Becker Underwood, US); M. anisopliae var acridum isolate IMI 330189 (ARSEF7486) (available as Green Muscle by Biological Control Products); Nomuraea rileyi; Paecilomyces fumosoroseus (new: Isaria fumosorosea), strain apopka 97 (available as PreFeRal® WG from Biobest); Paecilomyces fumosoroseus (new: Isaria fumosorosea) strain FE 9901 (available as NoFly® from Natural Industries Inc., a Novozymes company); and *Beauveria brongniartii* (e.g. Beaupro from Andermatt Biocontrol AG).

In an even more preferred embodiment, fungal strains having an insecticidal effect are selected from Beauveria bassiana, in particular strain ATCC 74040 (available as Naturalis® from Intrachem Bio Italia), strain GHA (Accession No. ATCC74250) (available as BotaniGuard Es and Mycontrol-O from Laverlam International Corporation), strain ATP02 (Accession No. DSM 24665), strain CG 716 (available as BoveMax® from Novozymes); Paecilomyces fumosoroseus (new: Isaria fumosorosea), strain apopka 97 (available as PreFeRal® WG from Biobest) and strain FE 9901 (e.g. NoFly® from Natural Industries Inc., a Novozymes company); Lecanicillium lecanii (formerly known as Verticillium lecanii), conidia of strain KV01 (available as Mycotal® and Vertalec® from Koppert/Arysta), conidia of strain strain DAOM198499 or conidia of strain DAOM216596; Metarhizium anisopliae, strain F52 (DSM3884/ ATCC 90448) (available as BIO 1020 by Bayer CropScience and also available as Met52 by Novozymes); Nomuraea rileyi; Lecanicillium muscarium (formerly Verticillium lecanii), strain VE 6 / CABI(=IMI) 268317/ CBS102071/ ARSEF5128; and *Beauveria brongniartii* (e.g. Beaupro from Andermatt Biocontrol AG).

If the fungal strain producing spores has a nematicidal effect, it may be selected from
D2.3 *Paecilomyces lilacinus,* in particular spores of *P. lilacinus* strain 251 (AGAL 89/030550; e.g. BioAct from Prophyta); D2.4 *Trichoderma koningii*; D2.5 *Harposporium anguillullae*; D2.6 *Hirsutella minnesotensis*; D2.7 *Monacrosporium cionopagum*; D2.8 *Monacrosporium psychrophilum*; D2.9 *Myrothecium verrucaria,* strain AARC-0255 (e.g. DiTeraTM by Valent Biosciences); D2.10 *Paecilomyces lilacinus ,* in particular spores of *P. lilacinus* strain 251 (AGAL 89/030550) (available as BioAct from Prophyta); D2.11 *Paecilomyces variotii,* strain Q-09 (e.g. Nemaquim® from Quimia, MX); D2.13 *Stagonospora phaseoli* (e.g. from Syngenta); D2.14 *Trichoderma lignorum,* in particular strain TL-0601 (e.g. Mycotric from Futureco Bioscience, ES); D2.15 *Fusarium solani,* strain Fs5; D2.16 *Hirsutella rhossiliensis;* D2.17 *Monacrosporium drechsleri*; D2.18 *Monacrosporium gephyropagum*; D2.19 *Nematoctonus geogenius*; D2.20 *Nematoctonus leiosporus*; D2.22 *Paraglomus sp,* in particular P. *Brasilianum*; D2.23 *Pochonia chlamydosporia* (also known as *Vercillium chlamydosporium*), in particular *var. catenulata* (IMI SD 187; e.g. KlamiC from The National Center of Animal and Plant Health (CENSA), CU); D2.24 *Stagonospora heteroderae*; D2.25 *Meristacrum asterospermum*; and D2.27 *Duddingtonia flagrans.*

In a more preferred embodiment, fungal strains with nematicidal effect are selected from
*Paecilomyces lilacinus,* in particular spores of *P. lilacinus* strain 251 (AGAL 89/030550) (available as BioAct from Prophyta); *Harposporium anguillullae*; *Hirsutella minnesotensis*; *Monacrosporium cionopagum*; *Monacrosporium psychrophilum*; *Myrothecium verrucaria*, strain AARC-0255 (available as DiTeraTM by Valent Biosciences); and *Paecilomyces variotii*; *Stagonospora phaseoli* (commercially available from Syngenta).

In an even more preferred embodiment, fungal strains with nematicidal effect are selected from *Paecilomyces lilacinus,* in particular spores of *P. lilacinus* strain 251 (AGAL 89/030550) (available as BioAct from Prophyta); and *Duddingtonia flagrans.*

If the fungal strain producing spores supports and/or promotes and/or stimulates plant health and plant growth it may be selected from
E2.1 *Talaromyces flavus,* in particular strain VII7b; E2.2 *Trichoderma atroviride,* e. g. strain no. V08/002387; E2.3 *Trichoderma atroviride,* strain no. NMI No. V08/002388; E2.4 *Trichoderma atroviride,* strain no. NMI No. V08/002389; E2.5 *Trichoderma atroviride,* strain no. NMI No. V08/002390; E2.6 *Trichoderma harzianum*, strain ITEM 908 (e.g. Trianum-P from Koppert); E2.7 *Myrothecium verrucaria,* strain AARC-0255 (e.g. DiTeraTM from Valent Biosciences); E2.8 *Penicillium bilaii,* strain ATCC 22348 (e.g. JumpStart® from Novozymes); E2.11 *Pythium oligandrum,* strain DV74 or M1 (ATCC 38472; e.g. Polyversum from Bioprepraty, CZ); E2.14 *Trichoderma atroviride,* strain LC52 (e.g. Sentinel from Agrimm Technologies Limited); E2.15 *Trichoderma harzianum*, strain TSTh20; E2.16 *Trichoderma koningii;* E2.31 *Trichoderma harzianum*, strain KD (e.g. Eco-T from Plant Health Products, SZ); E2.32 *Trichoderma harzianum*, strain 1295-22; E2.33 *Trichoderma virens,* strain GL-21; E2.34 *Verticillium albo-atrum* (formerly *V. dahliae*), strain WCS850 (CBS 276.92; e.g. Dutch Trig from Tree Care Innovations); and E2.35 *Trichoderma atroviride,* strain LC52 (e.g. Tenet from Agrimm Technologies Limited).

In a more preferred embodiment, fungal strains having a beneficial effect on plant health and/or growth are selected from
*Talaromyces flavus,*strain VII7b; ; *Myrothecium verrucaria*, strain AARC-0255 (available as DiTeraTM from Valent Biosciences); *Penicillium bilaii,* strain ATCC 22348 (available as JumpStart® from Novozymes); *Penicillium bilaii,* in particular strain ATCC 22348 (available as PB-50 PROVIDE from Philom Bios Inc., Saskatoon, Saskatchewan); and *Pythium oligandrum,* strain DV74 or M1 (ATCC 38472) (available as Polyversum from Bioprepraty, CZ);.

In an even more preferred embodiment, fungal strains having a beneficial effect on plant health and/or growth are selected from *Penicillium bilaii,* in particular strain ATCC 22348 (available as JumpStart® from Novozymes) and strain ATCC 22348 (available as PB-50 PROVIDE from Philom Bios Inc., Saskatoon, Saskatchewan).

If the fungal strain producing spores has a herbicidal effect it may be selected from
F2.1 *Phoma macrostroma,* strain 94-44B (e.g. Phoma H and Phoma P by Scotts, US); F2.3*Colletotrichum gloeosporioides,* strain ATCC 20358 (e.g. Collego (also known as LockDown) by Agricultural Research Initiatives); and F2.4 *Stagonospora atriplicis.*

In a more preferred embodiment, said fungal spores are from Paecilomyces lilacinus (recently re-classified as Purpureocillium lilacinum). A number of Paecilomyces lilacinus strains have been described for use as a biological control agent. Such strains include strain 251 in the products BioAct, MeloCon and NemOut produced by Prophyta GmbH, a strain 580 in the product Biostat WP (ATCC no. 38740) produced by Laverlam, a strain in the product Bio-Nematon produced by the company T.Stanes and Company Ltd., a strain in the product Mysis produced by the company Varsha Bioscience and Technology India Pvt Ltd., one in the product Bioiconema available from Nico Orgo Maures, India, one in the product Nemat, available from Ballagro Agro Tecnologia Ltda, Brazil and one in the product Spectrum Pae L available from Promotora Tecnica Industrial, S.A. DE C.V., Mexico.

In an even more preferred embodiment said Paecilomyces lilacinus is Paecilomyces lilacinus strain 251 as described in WO1991/002051 or a mutant thereof having all identifying characteristics of the respective strain.

Preferred spore concentrations in a formulation according to the invention for nematode control using Purpureum lilacinum, in particular P. lilacinum strain 251, range between 5 x 10⁹ and 1 x 10¹¹ spores/ml, preferably at least 4 x 10¹⁰, more preferably at least 5 x 10¹⁰ spores/ml.
It is further preferred that in a formulation with Paecilomyces lilacinus, in particular P. lilacinus strain 251, as biological control agent, the concentration of fumed silica or precipitated silica ranges between 3.5 and 6% wt, such as between 4 and 5.5% wt, e.g. 4.4, 4.6, 4.8, 5.0 or 5.2% wt.

The spore-producing fungal strain is cultivated according to methods known in the art, such as liquid or solid fermentation, or as described elsewhere in this application on an appropriate substrate, e. g. by submerged fermentation or solid-state fermentation, e. g. using a device disclosed in WO2005/012478 or WO1999/057239. Subsequently, the microorganism or its organs used as biological control agent is/are separated from the substrate. The substrate populated with the microorganism is dried preferably before the separation step. The microorganism or its organs may be dried via e. g. freeze-drying, vacuum drying or spray drying after separation. Where necessary, during the drying process the spore containing biomass is combined through blending, spraying, mixing, and/or extruding with one or more inert solid carriers that can include, but are not limited to, clay, bran, lactose, cellulose, vermiculite, skim milk or sawdust. After separation and drying, the microorganism or its organs is/are suspended in the carrier according to the invention comprising fumed silica or precipitated silica and a polyether-modified trisiloxane as described herein in order to form the liquid formulation which is then packaged according to the method of the invention.

In one embodiment, said spores are of an entomopathogenic fungus.

The utility of the method is not dependent upon the number of spores in the formulation. However, a typical formulation includes 40% by weight or less spore-containing biomass. Accordingly, a formulation, depending on application and species of the fungal spores, may comprise 30%, 25%, 20%, 15%, 10% or even only 5% spore containing biomass. Depending on the mode of preparation, i.e. production of the dried spores with or without addition of powder adjuvants, the percentage of spores in the total solid mass may vary as well..

Moreover, the storage and use conditions can vary with the particular application.
The oxygen available to the spores of the spore-based biocontrol formulation is reduced by placing the product into a closed and sealable container as described elsewhere, and preventing oxygen in the container from being or becoming available to the product.

The sealable container is formed of any material through which air cannot pass. Preferably, the container is formed of a plastic material with which an air-tight seal can be made using a standard heat-sealing process or using such other adhesive sealing process as is known to the art. The container may be transparent, translucent or opaque and may be formed of a single piece of material or more than one piece of material, wherein all edges of the container are sealed to prevent entry of outside air. The container can optionally include a one-way valve which permits gas to leave the interior of the container without permitting outside air to enter. The container may also include appropriate identifying indicia describing the contents and other useful data. The indicia may be provided directly on the container surface or on a label affixed thereto.

The present invention furthermore relates to a method of combatting plant pests or phytopathogenic fungi comprising providing the container according to the invention, preparing the formulation contained therein for agricultural use or for use as a biocide and applying said prepared formulation to a plant or a spot in need thereof.

Finally, the present invention relates to the use of the container according to the invention in agriculture or as a biocide.

The examples illustrate the invention.

### Example 1: Comparison of packaging materials allowing for oxygen diffusion or being airtight

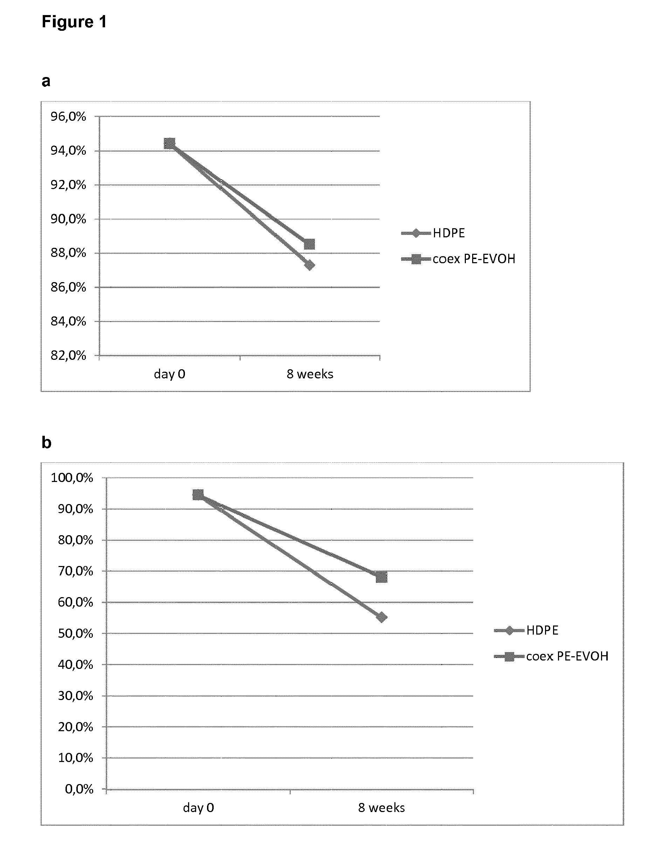

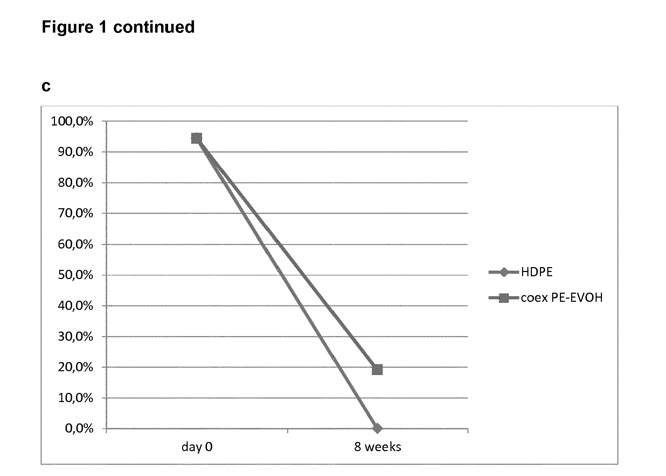

100mL of the formulation comprising a polyether-modified trisiloxane, fumed silica and spores of Purpureocillium lilacinum were filled in bottles (HDPE or Coex(PE/EVOH) respectively, nominal filling volume 100 mL) and sealed. The sealed bottles were then stored in temperature controlled storage cabinets (4°C, 30°C and 40°C) for a defined time. Before determination of viability, the bottles were equilibrated to ambient temperature. The viability was determined using a microbiological counting method. The values listed in the following tables are illustrated in Figures 1a to 1c.

### Example 1a:

| Storage temperature: 4 °C | | |
|---|---|---|
| Sample taken at/after | HDPE | Coex (PE/EVOH) |
| day 0 | 94,4% | 94,4% |
| 8 weeks | 87,3% | 88,5% |

### Example 1b:

| Storage temperature: 30 °C | | |
|---|---|---|
| Sample taken at/after | HDPE | Coex (PE/EVOH) |
| day 0 | 94,4% | 94,4% |
| 8 weeks | 55,2% | 68,1% |

### Example 1c:

| Storage temperature: 40 °C | | |
|---|---|---|
| Sample taken at/after | HDPE | Coex (PE/EVOH) |
| day 0 | 94,4% | 94,4% |
| 8 weeks | 0,0% | 19,1% |

### Example 2: Comparison of sealed bottles with atmospheric and reduced oxygen content

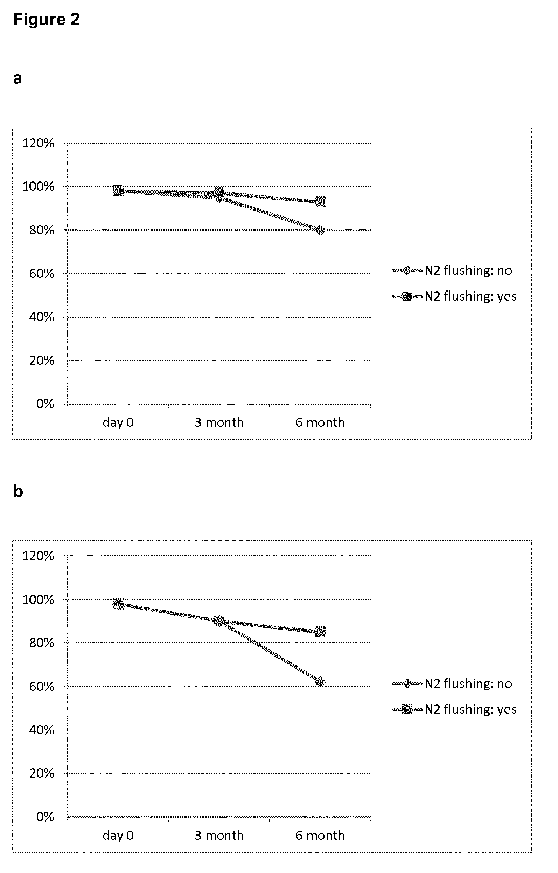

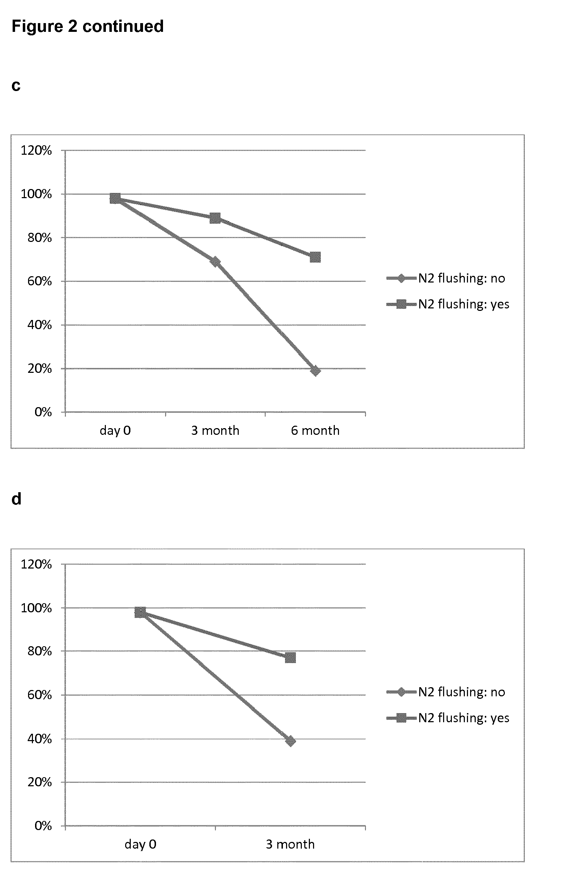

100mL of the formulation comprising a polyether-modified trisiloxane, fumed silica and spores of Purpureocillium lilacinum were filled in coextruded bottles (PE/EVOH, nominal filling volume 100 mL) and sealed with or without prior nitrogen flushing. In case of directly sealing the bottle, the initial oxygen content in the headspace of the bottle reflects the atmospheric oxygen content of ca. 21%. In the case of flushing the filled bottle with nitrogen prior to sealing, the oxygen content was reduced by at least 50%. This was established by pre-trial where filled bottles where flushed with nitrogen and sealed and the oxygen content of the sealed bottle immediately determined by use of an oxygen sensor. The sealed bottles were

then stored in temperature controlled storage cabinets (20°C, 25°C, 30°C and 35°C) for a defined time. Before determination of viability, the bottles were equilibrated to ambient temperature. The viability was determined using a microbiological counting method. The values listed in the following tables are illustrated in Figures 2a to 2d.

### Example 2a:

| Storage temperature: 20 °C | | |
|---|---|---|
| Sample taken at/after | N2 flushing: no | N2 flushing: yes |
| day 0 | 98% | 98% |
| 3 month | 95% | 97% |
| 6 month | 80% | 93% |

### Example 2b:

| Storage temperature: 25 °C | | |
|---|---|---|
| Sample taken at/after | N2 flushing: no | N2 flushing: yes |
| day 0 | 98% | 98% |
| 3 month | 90% | 90% |
| 6 month | 62% | 85% |

### Example 2c:

| Storage temperature: 30 °C | | |
|---|---|---|
| Sample taken at/after | N2 flushing: no | N2 flushing: yes |
| day 0 | 98% | 98% |
| 3 month | 69% | 89% |
| 6 month | 19% | 71% |

### Example 2d:

| Storage temperature: 35°C | | |
|---|---|---|
| Sample taken at/after | N2 flushing: no | N2 flushing: yes |
| day 0 | 98% | 98% |
| 3 month | 39% | 77% |

## Claims

1. A method for prolonging spore viability of fungal spores present in a liquid formulation, comprising
(a) packaging said formulation in a suitable container;
(b) reducing oxygen exposure of the spores in said formulation as compared to oxygen exposure of said spores when said formulation is in contact with air; and
(c) closing or sealing said container.

2. The method of claim 1, wherein said container is with reduced or without head space.

3. The method of claim 1 or 2, wherein said container reduces or prevents diffusion of oxygen.

4. The method of any one of claims 1 to 3, wherein said reducing oxygen exposure in step (b) is effected by reducing oxygen in the head space of said container.

5. The method of any one of claims 1 to 4, wherein reducing oxygen exposure in step (b) is effected by evacuating said container.

6. The method of any one of claims 1 to 5, wherein reducing oxygen exposure in step (b) is effected by evacuating said container and subsequent refilling with an (inert) gas or mixtures of (inert) gases.

7. The method of claim 6, wherein step (b) is repeated at least twice.

8. The method of claim 4, wherein said oxygen exposure in step (b) is reduced by flushing said head space with an (inert) gas or a mixture of gases comprising less oxygen compared to air or no oxygen.

9. The method of any one of claims 6 to 8, wherein said gas is hydrogen, nitrogen, helium, neon, argon, krypton, xenon, radon, carbon dioxide, nitrous oxide, hydrogen sulfide, lower alkane or halo alkane or mixtures thereof.

10. The method of any one of claims 6 to 9, wherein said gas is nitrogen.

11. The method of any one of claims 1 to 10, wherein said formulation is essentially free of water.

12. A closed container comprising a liquid formulation within said container, said formulation comprising fungal spores, wherein the spores in said formulation have reduced oxygen exposure as compared to oxygen exposure of said spores when said formulation is in contact with air.

13. The container of claim 11 or 12, wherein said spores are from a fungal species selected from Purpureocillium lilacinum, Isaria fumosorosea, Beauveria bassiana, Cladosporium cladosporioides, Clonostachys rosea, Coniothyrium minitans, Metarhizium anisopliae, Nomurea rileyi, Penicillium bilaii, Simplicillium lanosoniveum and Talaromyces flavus.

14. The container of any one of claims 11 to 13, wherein said spores are from Purpureocillium lilacinum.

15. The container of claim 14, wherein said Purpureocillium lilacinum is strain 251.

16. The container of any one of claims 11 to 15, wherein said formulation is essentially free of water.

17. Method of combatting plant pests or phytopathogenic fungi comprising providing the container according to any one of claims 11 to 16, preparing the formulation contained therein for agricultural use or for use as a biocide and applying said prepared formulation to a plant or a spot in need thereof.

18. Use of the formulation comprised in the container of any one of claims 11 to 16 in agriculture or as a biocide.
